**Europäisches Patentamt**

**European Patent Office**

**Office européen des brevets**

(19)

(11) Veröffentlichungsnummer: **0 019 792**
**B1**

(12) # EUROPÄISCHE PATENTSCHRIFT

(45) Veröffentlichungstag der Patentschrift:
**06.04.83**

(21) Anmeldenummer: **80102636.0**

(22) Anmeldetag: **13.05.80**

(51) Int. Cl.³: **G 02 B 21/22**, A 61 B 1/00,
G 02 B 23/00

(54) **Zusatzobjektive für Operationsmikroskope.**

(30) Priorität: **16.05.79 DE 2919678**

(43) Veröffentlichungstag der Anmeldung:
**10.12.80 Patentblatt 80/25**

(45) Bekanntmachung des Hinweises auf die Patenterteilung:
**06.04.83 Patentblatt 83/14**

(84) Benannte Vertragsstaaten:
**CH FR GB LI**

(56) Entgegenhaltungen:

**DE-A-2 309 203**
**DE-A-2 556 717**
**DE-B-1 772 098**
**DE-B-1 772 824**
**US-A-4 061 135**

(73) Patentinhaber: **Firma Carl Zeiss, Postfach 1369/1380,
D-7082 Oberkochen (DE)**

(72) Erfinder: **Lang, Walter H., Finkenweg 33,
D-7923 Königsbromm (DE)**
Erfinder: **Muchel, Franz, Dipl.-Phys., Lortzingstrasse 2,
D-7923 Königsbronn (DE)**

Zusatzobjektive für Operationsmikroskope

Die Erfindung betrifft Zusatzobjektive für binokulare Operationsmikroskope, die insbesondere für Eingriffe in engen und tiefen Operationskanälen geeignet sind.

Bei Operationsmikroskopen handelt es sich um Stereomikroskope, die für mikrochirurgische Eingriffe in geeigneter Weise an Tragarmen und Kupplungen gehaltert sind. Diese Stereomikroskope besitzen Wechselobjektive mit unterschiedlicher Brennweite, die meist als einziges Linsenglied mit grossem Durchmesser ausgeführt sind, das von den optischen Achsen beider Teilstrahlengänge exzentrisch durchsetzt ist. Ein Stereomikroskop dieses Typs ist beispielsweise in der US-A-3 704 932 beschrieben.

Damit der Operateur bei der Führung seiner mikrochirurgischen Instrumente durch das Mikroskop nicht behindert wird, muss ein minimaler freier Arbeitsabstand der Operationsmikroskopvorderfläche vom Operationsfeld gefordert werden. Er beträgt je nach Fachrichtung und je nach dem durchzuführenden operativen Eingriff zwischen 100 und 400 mm. Zur ungestörten Beobachtung und Beleuchtung muss ein relativ grosser kegelförmiger Bereich, in dem die Strahlengänge verlaufen, vor dem Objektiv freigehalten werden.

Für Eingriffe in engen und tiefen Operationskanälen wie sie zum Beispiel bei der Mikro-Chirurgie an den Stimmbändern oder an Bandscheiben gang und gäbe sind, kann es jedoch vorkommen, dass im Operationskanal und wegen der eingeführten mikro-chirurgischen Instrumente der Beleuchtungsstahlengang teilweise beschnitten wird. Dadurch wird die verfügbare Beleuchtung des Operationsfeldes herabgesetzt. Ungleich schwerer wiegt jedoch die Tatsache, dass beide Beobachtungsstrahlengänge zum Teil abgedeckt werden oder gar im Extremfall nur ein einziger Beobachtungsstrahlengang benutzt werden kann. Dadurch entfällt eine der wichtigsten Voraussetzungen für den Operateur, nämlich eine hinreichend gute Stereobeobachtung und damit Tiefenlokalisation im Operationsbereich für die Führung der mikro-chirurgischen Instrumente.

Ein radiales Verkleinern des Beleuchtungs- bzw. Beobachtungskegels duch Vergrössern des Arbeitsabstandes verbietet sich im allgemeinen wegen der damit verbundenen Verringerung des Konvergenzwinkels, der im Interesse guter Stereobeobachtung möglichst gross sein sollte, und der ohnehin bei Operationsmikroskopen recht niedrigen Apertur der Objektive.

Es ist aus der DE-A-2 309 203 bekannt, eine kegelförmige Umkleidung des für den Strahlengang freizuhaltenden Bereichs vorzusehen. Diese rein mechanische Massnahme verhindert zwar eine Abschattung der Strahlengänge durch chirurgische Instrumente, vergrössert jedoch nicht den für Manipulation benötigten Raum. Zum Einführen in lange, zylindrische Öffnungen ist ein mit einer derartigen Umkleidung versehenes Objektiv nicht geeignet. Bezüglich des Konvergenzwinkels und der Apertur gilt das vorstehend Gesagte.

Allgemein werden zu mikroskopischen Beobachtungen in tiefen bzw. engen Körperöffnungen starre oder flexible Endoskope benutzt. Es sind jedoch relativ wenige Endoskope bekannt, die eine stereoskopische Beobachtung ermöglichen.

In der DE-C-2 454 370 ist ein derartiges Endoskop beschrieben, das aus zwei mit einer Klammer gekoppelten flexiblen Einzelendoskopen besteht, das jedoch nur zur Einführung in Nasenlöcher geeignet ist. Die US-A-3 520 587 beschreibt ebenfalls ein flexibles Stereoendoskop, ds in einem Hüllrohr zwei separate Bildleiterfaserbündel enthält.

Flexible Endoskope sind jedoch nur schlecht zur Beoabachtung von Operationsfeldern geeignet, da ihre Bildgüte selbst bei sehr guten Bildleitern bedingt durch die Struktur der Einzelfaseranordnungen sowie angebrochene oder vollständig gebrochene Fasern grundsätzlich schlechter als die von Operationsmikroskopen ist. Ihr Vorteil der Flexibilität wird zudem bei der Operationsmikroskopie nicht benötigt, er kann sich im Gegenteil durch Bildinstabilitäten nachteilig auswirken.

Aus der DE-A-1 766 803, dem DE-U-1 996 605 sowie der US-A-4 061 135 sind starre Endoskope bekannt. Diese besitzen einen langgestreckten Grundkörper, in dem zwei parallele Optiken angeordnet sind und dessen proxymales Emde einen Stereovorsatz zur binokularen Beobachtung oder zur Stereofotographie trägt. Die doppelte Auslegung des Beobachtungskanals und die damit verbundenen zusätzlichen Zentrierarbeiten innerhalb der Kanäle sowie beider Kanäle zueinander machen diese Instrumente recht aufwendig in der Herstellung.

Zudem besitzen alle Endoskope den Nachteil, dass sie entsprechend ihrer Aufgabe vom Endoskopiker mit der Hand zu führen sind, so dass nur noch eine Hand für die Bedienung der Einstellelemente und die Handhabung chirurgischer Instrumente bleibt. Bei Anschluss einer Fernsehkamera oder eines Mitbeobachtertubus, wie das bei Operationsmikroskopen häufig der Fall ist, würde ein Endoskop aus den vorstehend genannten Gründen nur noch sehr schwer bedienbar sein.

Es ist die Aufgabe der vorliegenden Erfindung, den Anwendungsbereich eines herkömmlichen binokularen Operationsmikroskops dahingehend zu erweitern, dass Eingriffe in engen Körperhöhlen ohne Beeinträchtigung des stereoskopischen Sehvermögens ausgeführt werden können.

Diese Aufgabe wird durch Zusatzobjektive gelöst, die gemäss dem kennzeichnenden Teil des Hauptanspruchs ausgebildet sind.

Damit ist es dem Benutzer möglich, sein bereits vorhandenes Operationsmikroskop ohne komplizierte Umbauten lediglich durch Ansetzen des Zusatzobjektivs für Eingriffe in engen Körperhöhlen umzurüsten. Bedingt durch die Form der Zusatzobjektive ist der zur Führung der Beobachtungsstrahlengänge nötige Raumbedarf minimiert und gegen unbeabsichtigtes Abschatten beim Operieren geschützt. Der für das räumliche Sehen wichtige Konvergenzwinkel und die für Auflösung und Bildhelligkeit massgebliche Apertur verringern sich nicht, da die Verringerung der Beobachtungsbasis bzw. des Objektivdurchmessers durch die kürzere Brennweite bzw. den geringeren Objektabstand der Zusatzobjektive kompensiert wird.

Vorteilhaft ist es, im Objektivgehäuse Kanäle zur Durchführung chirurgischer Instrumente und zur Spülung des Operationsraumes vorzusehen, da durch die erste Massnahme der Patient geschützt wird und dem Operateur die Führung langer Bestecke erleichtert wird, und durch die zweite Massnahme freie Sicht auf das zu behandelnde Objekt erzielt wird.

Durch die Verwendung eines Sichtkanals zur Übertragung beider stereoskopischer Strahlengänge sind derartige Objektive leicht und billig herzustellen. Da mehrere Wechselobjektive mit unterschiedlichen Baulängen und Abbildungsmassstäben vorgesehen sind, ist das damit ausgerüstete Operationsmikroskop universell verwendbar.

Besonders ökonomisch lassen sich die Wechselobjektive einsetzen, wenn mehrere von ihnen sich zu einem längeren Objektiv zusammensetzen lassen. Beispielsweise ist es sinnvoll, mehrere Teilobjektive unterschiedlicher Baulänge mit einem konstanten Abbildungsmassstab und mehrere Teilobjektive konstanter, kurzer Baulänge mit unterschiedlichen Abbildungsmassstäben vorzusehen, um eine universelle Verwendung des Operationsmikroskops bei möglichst wenigen Zubehörteilen zu ermöglichen.

Die Erfindung wird anhand der Figuren 1 bis 5 der beigefügten Zeichnungen beispielsweise näher erläutert.

Fig. 1 zeigt schematisch einen Schnitt durch ein Ausführungsbeispiel eines erfindungsgemässen Zusatzobjektivs an einem Operationsmikroskop;

Fig. 2a zeigt ein auswechselbares Zwischenstück für das in Fig. 1 dargestellte Operationsmikroskop;

Fig. 2b zeigt ein herkömmliches Wechselobjektiv für ein Operationsmikroskop;

Fig. 3 zeigt ein erfindungsgemässes Wechselobjektiv für ein Operationsmikroskop;

Fig. 4 zeigt die Verwendung der in Fig. 1 dargestellten Wechselobjektive als Endoskop;

Fig. 5 zeigt einen Querschnitt durch ein Wechselobjektiv.

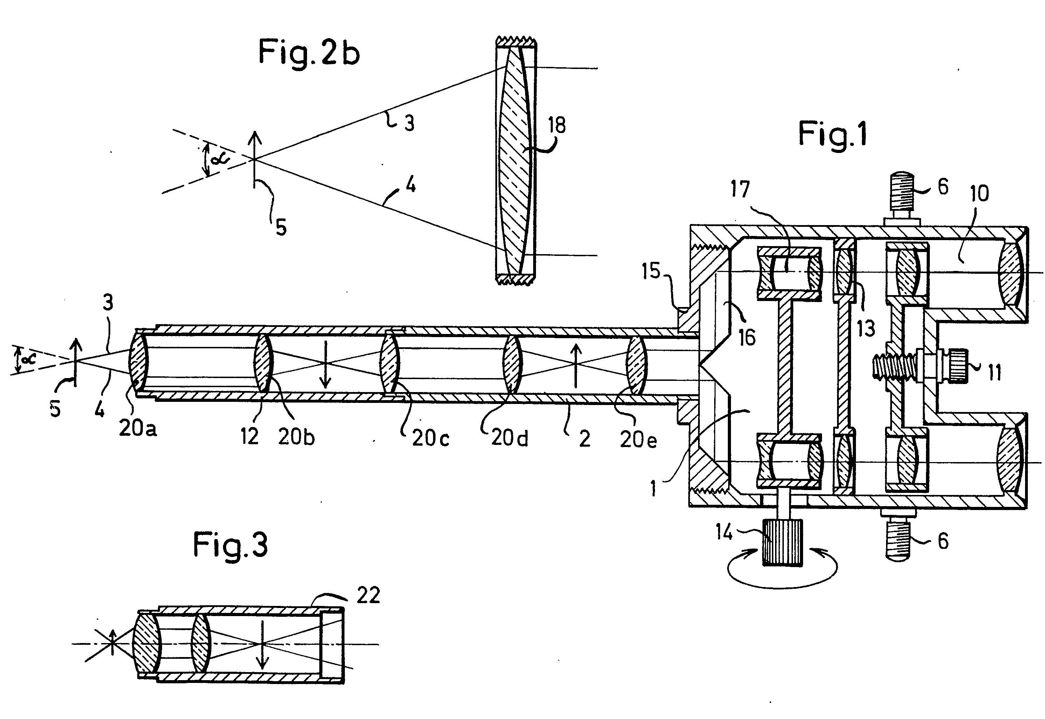

Das in Fig. 1 dargestellte Operationsmikroskop besteht aus einem Gehäuse 1, das mittels zweier Gewindebolzen 6 an nicht dargestellten Tragarmen kipp- und schwenkbar gehaltert wird. Das Gehäuse 1 enthält die für stereoskopisches Sehen nötige Optik bestehend aus den Okularen 10, die jeweils ein über einen gemeinsamen Feintrieb 11 axial verstellbares Linsenglied enthalten, mit dem das von den Tubuslinsen 13 erzeugte Zwischenbild kontinuierlich nachvergrössert werden kann. Ein Wechseln des Abbildungsmassstabes in festen, grösseren Schritten ist mit Hilfe des Revolvers 14 möglich, der das Einschwenken verschiedener Linsenkombinationen 17 in die beiden Strahlengänge erlaubt.

Nicht dargestellt sind die bei Operationsmikroskopen üblichen Zusatzvorrichtungen zur Mitbeobachtung und Dokumentation. Dazu gehören zum Beispiel Strahlenteiler, um den Beobachtungsstrahlengang zweckentsprechend aufzuteilen, und Stutzen zum Anschluss einer Kleinbildkamera, einer Fernsehkamera und eines oder mehrerer Mitbeobachtertuben.

In der Frontpartie des Gehäuses 1 ist ein Zwischenstück 15 herausnehmbar befestigt, das zwei Prismen 16 enthält, mit dem die Beobachtungsbasis verkleinert wird, und an das ein zweiteiliges stabförmiges Wechselobjektiv 2, 12 angesetzt ist.

Das Zwischenstück 15 ist gegen das in Fig. 2b dargestellte Wechselobjektiv 18 austauschbar. Nach Einschrauben des Objektivs 18 in das Gehäuse 1 entsteht ein Operationsmikroskop herkömmlicher Bauart, das zum Beispiel vorteilhaft zur Untersuchung grösserer ebener Objektfelder eingesetzt werden kann (Dermatologie) also in Fällen, bei denen die stabförmigen Wechselobjektive nicht benötigt werden.

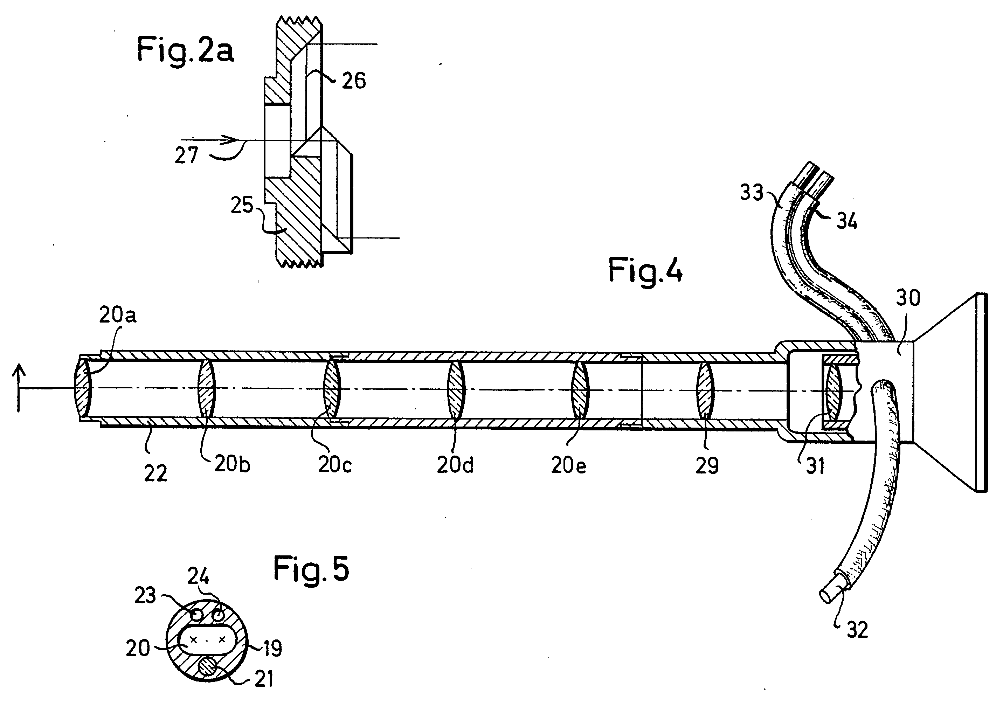

Das Zwischenstück 15 ist aber auch gegen das in Fig. 2a dargestellte Zwischenstück 25 austauschbar, in dem ein Prismensystem 26 so angeordnet ist, dass eine Aufteilung des Strahlenganges 27 für binokulare Beobachtung erfolgt. Mit Hilfe dieses Zwischenstückes 25, an das die Wechselobjektive 2, 22 ebenfalls angesetzt werden können, ist das Operationsmikroskop auch in Fällen einsetzbar, in denen es weniger auf gute Stereobeobachtung als vielmehr auf hohe Auflösung flächiger Objekte in Körperhöhlen ankommt.

Die in beiden Fällen verwendbaren Wechselobjektive 2, 12, 22 haben die Form langgestreckter Zylinder mit kreisförmigem Querschnitt. Sie enthalten neben dem in Fig. 1 dargestellten Linsensystem für die Abbildung Kanäle 23, 24, durch die Flüssigkeiten zur Spülung des Objektraumes geleitet werden können (Fig. 5). Ausserdem ist in den Objektiven ein Glasfaserstab 21 angeordnet, über den der Objektraum beleuchtet werden kann.

Die das Objekt mehrfach zwischenabbildenden Linsenglieder 20a-20e besitzen einen ovalen Querschnitt, wodurch ein vertretbarer Kompromiss zwischen der Forderung nach möglichst geringem Aussendurchmesser der Objekte und möglichst grossem Konvergenzwinkel für die Stereobeobachtung erreicht wird.

Anstelle des Objektivs 12 kann auch das in Fig. 3 dargestellte Objektiv 22 eingesetzt werden. Dieses besitzt neben kürzerer Bauform einen grösseren Abbildungsmassstab. Natürlich kann das in Fig. 1 dargestellte Operationsmikroskop auch ohne die Objektive 12 und 22 lediglich unter Verwendung des Objektivs 2 benutzt werden. Nach der Entfernung des Objektivs 12 tritt zwar eine Bildumkehrung ein, die jedoch dadurch vermieden werden kann, dass die auszuwechselnden Objektive immer für positiven Abbildungsmassstab konzipiert werden, oder dass zusätzliche bildumkehrende Mittel schaltbar im Tubus 1 angeordnet werden.

Fig. 4 zeigt die Verwendung der Objektive 2, 22 als Endoskop. Sie werden zu diesem Zwecke mit einem Tubus 30 verbunden, der eine Linse 29 zur Zwischenabbildung des Objekts und ein Variookular 31 enthält. Die Schläuche 33 und 34 sind mit den Kanälen 23 und 24 in den Objektiven verbunden. Zur Beleuchtung des Objekts ist das flexibel herausgeführte Faserbündel 32 an geeignete Lichtquellen anzuschliessen.

Um dem Benutzer die Führung des Endoskops zu erleichtern, kann das Instrument an Tragarmen leicht beweglich gehaltert sein. Im Tubus 30 können Mittel zur Strahlteilung und zum Anschluss von Mitbeobachtungs- bzw. Dokumentationssystemen vorgesehen sein.

**Patentansprüche**

1. Zusatzobjektive für binokulare Operationsmikroskope mit einem auswechselbaren, langbrennweiten Objektiv zur Führung beider stereoskopischer Strahlengänge, dadurch gekennzeichnet, dass die Zusatzobjektive (2, 12, 22) starr und stabförmig mit im Vergleich zu dem langbrennweitigen Objektiv (18) kleinem Durchmesser und kurzer Brennweite ausgebildet sind, und mittels eines Zwischenstückes (15), das eine Aufnahme für die stabförmigen Objektive (2, 12, 22) und eine Optik (16) zur Verkleinerung der Beobachtungsbasis aufweist, anstelle des langbrennweitigen Objektivs (18) in das Operationsmikroskop einsetzbar sind.

2. Zusatzobjektive nach Anspruch 1, dadurch gekennzeichnet, dass sie ein Relaislinsensystem (20) zur Übertragung der beiden für stereoskopisches Sehen nötigen Beobachtungsstrahlengänge (3, 4) aufweisen.

3. Zusatzobjektive nach Anspruch 1 oder 2, dadurch gekennzeichnet, dass sie zwischen Linsen (20) und Fassung (19) axial verlaufende Kanäle (21, 23, 24) aufweisen, durch die der Beleuchtungsstrahlengang, Spülflüssigkeiten und/oder mikro-chirurgische Instrumente zum Objektraum geführt werden können.

4. Zusatzobjektive nach Anspruch 2 oder 3, dadurch gekennzeichnet, dass sie unterschiedliche Baulängen aufweisen.

5. Zusatzobjektive nach Anspruch 4, dadurch gekennzeichnet, dass mehrere Objektive (2, 12, 22) kurzer Baulänge zu einem Zusatzobjektiv grosser Baulänge verbindbar sind.

6. Zusatzobjektive nach einem der Ansprüche 1 bis 5, dadurch gekennzeichnet, dass sie unterschiedlichen Arbeitsabstand und Abbildungsmassstab aufweisen.

7. Zusatzobjektive nach einem der Ansprüche 1 bis 6, dadurch gekennzeichnet, dass das Zwischenstück (15) mit Prismen zur Verkleinerung der stereoskopischen Beobachtungsbasis gegen ein Zwischenstück (25) auswechselbar ist, das Prismen zur Strahlteilung für reine Binokularbeobachtung aufweist.

**Revendications**

1. Objectifs supplémentaires pour microscopes opératoires binoculaires, avec un objectif interchangeable à grande distance focale destiné au guidage des deux faisceaux stéréoscopiques, caractérisé en ce que les objectifs supplémentaires (2, 12, 22) sont construits rigides et en forme de cylindre, avec un diamètre plus petit et une focale plus courte que l'objectif à grande focale (18), et qu'ils peuvent être installés dans le microscope opératoire, à la place de l'objectif à grande focale (18), par l'intermédiaire d'un adaptateur (15), lequel possède un évidement destiné aux objectifs (2, 12, 22) en forme de cylindre et à une optique (16) servant à la réduction de la base d'observation.

2. Objectifs supplémentaires selon la revendication 1, caractérisé en ce qu'ils possèdent un système de lentilles de relais (20) destiné au transfert des deux faisceaux d'observation (3, 4) nécessaires à la vision stéréoscopique.

3. Objectifs supplémentaires selon l'une quelconque des revendications 1 ou 2, caractérisé en ce qu'ils possèdent entre les lentilles (20) et la monture (19) des conduits axiaux (21, 23, 24), à travers lesquels on peut faire passer le faisceau d'éclairement, des liquides de nettoyage et/ou des instruments de micro-chirurgie, jusqu'à l'espace objet.

4. Objectifs supplémentaires selon l'une quelconque des revendications 2 ou 3, caractérisé en ce qu'ils ont des longueurs mécaniques différentes.

5. Objectifs supplémentaires selon la revendication 4, caractérisé en ce que plusieurs objectifs (2, 12, 22) de faible longueur mécanique peuvent être assemblés pour donner un objectifs supplémentaire de grande longueur mécanique.

6. Objectifs supplémentaires selon l'une quelconque des revendications 1 à 5, caractérisé en ce qu'ils ont différentes distances de travail et différents grandissements.

7. Objectifs supplémentaires selon l'une quelconque des revendications 1 à 6, caractérisé en ce que l'adaptateur (15), muni de prismes destinés à la réduction de la base d'observation stéréoscopique, peut être remplacé par un adaptateur (25), lequel contient des prismes destinés à la division di faisceau, pour assurer une observation binoculaire propement dite.

**Claims**

1. Additional objectives for binocular operation microscopes, with an exchangeable objective of long focal length conducting both stereoscopic ray paths, characterized by the fact that the additional objectives (2, 12, 22) are formed rigid and bar shaped having a small diameter and a short focal length compared to the objective (18) of long focal length, and are connectable to the operation microscope instead of the objective with long focal length by use of an intermediate member 15 which contains an adapter for the bar shaped objectives (2, 12, 22) and an optical system (16) for decreasing of the stereoscopic observation base.

2. Additional objectives according to claim 1, characterized by the fact that they contain one relay lens system (20) for the transmission of both observation ray paths (3, 4) necessary for stereoscopic viewing.

3. Additional objectives according to claims 1 or 2, characterized by the fact that they contain axially extending channels (21, 23, 24) between the lenses (20) and the mount (19) through which channels the illuminating ray path, liquids for flushing and/or micro-surgical instruments can be conducted to the object.

4. Additional objectives according to claims 1 or 2, characterized by the fact that they have different structural lenghts.

5. Additional objectives according to claim 4, characterized by the fact that a plurality of interchangeable objectives (2, 12, 22) of shorter structural length can be combined to form an additional objective of large structural length.

6. Additional objectives according to one of the claims 1 to 5, characterized by the fact that they have different working distances and different linear magnifications.

7. Additional objectives according to one of the claims 1 to 6, characterized by the fact that the intermediate member (15) having prisms for decreasing the stereoscopic observation base can be replaced by an intermediate member (25) which contains prisms for pupil splitting for pure binocular observation.

Fig.2b

Fig.1

Fig.3

0 019 792

**Fig.2a**

26

27

25

**Fig.4**

33

34

20a

30

22

20b

20c

20d

20e

29

31

32

**Fig.5**

24

23

20

19

21

0 019 792